# EUROPEAN PATENT APPLICATION

(11) **EP 4 578 956 A1**
(43) Date of publication of application: **02.07.2025**
(21) Application number: 24222927.6
(22) Date of filing: 23.12.2024
(51) Int. Cl.: C12Q 1/04, C12Q 1/18

(54) **REAGENTS FOR DETECTION USING ENZYMATIC CYCLING REACTIONS**

(30) Priority: 27.12.2023 JP 2023221660
(71) Applicant: Kanto Kagaku Kabushiki Kaisha, Tokyo 103-0023 (JP)
(72) Inventor: UCHIDA, Norikuni, Kanagawa, 2591146 (JP); YAMAZAKI, Hiroyuki, Kanagawa, 2591146 (JP)
(74) Representative: Meissner Bolte Partnerschaft mbB

(57) **Abstract**

[Object] An object of the present invention is to provide reagents that can be used for drug susceptibility testing of a subject or for determining the presence or absence of the subject and that are versatile and tested by simple methods and are capable of rapidly providing results, which have not been realized by conventional techniques.

[Solution] The present invention relates to a reagent for detecting an intracellular detectable component in a sample, comprising a cell processing agent for transferring the intracellular detectable component to the outside the cell, and a composition for an enzyme cycling reaction for performing the enzyme cycling reaction to the detectable component, and a method for determining the susceptibility of a microbial cell to the cell processing agent or for determining the presence or absence of the microbial cell in a sample using the reagent.

## Description

### TECHNICAL FIELD

The present invention relates to reagents for detection using enzymatic cycling reactions and methods of detection using the same.

### BACKGROUND ART

In the treatment of infectious diseases caused by pathogens such as bacteria, hospitals and the like conduct both tests to identify the pathogen infecting the patient and tests to specify the antimicrobial agents that are effective against that pathogen.

In recent years, with respect to the identification of bacteria, an extremely rapid identification method using MALDI-TOF/MS, a mass spectrometer, has been implemented (Patent Literature 1).

On the one hand, there still is no rapid test method for specifying the antimicrobial agents that are effective against the bacterium. For this reason, there is often a case that an antimicrobial agent is administered based on experience. However, it has been reported that a significantly higher mortality rate is shown if the antimicrobial agent is inappropriate with respect to the drug susceptibility of the bacteria (Non-Patent Literature 1). In particular, it has been shown that septic patients have a survival rate of approximately 70% when appropriate antimicrobial agents are administered within 2 hours of onset, with survival rates declining by 7.6% every one hour thereafter (Non-Patent Literature 2).

As common methods for testing drug susceptibility, micro-liquid dilution method and disk diffusion method are widely used. In the micro-liquid dilution method, a certain amount of a bacterium is inoculated into media containing varying concentrations of an antimicrobial agent, and the growth of the bacterium is observed to determine the maximum concentration at which the bacterium could grow in order to determine the susceptibility of the bacterium to the antimicrobial agent. In the disk diffusion method, the bacterium is plated onto an agar medium and cultured with a disc containing an antimicrobial agent is placed thereon, and the susceptibility of the bacterium to the antimicrobial agent is determined based on the size of the inhibition circle formed around the disc (Non-Patent Literature 3). These methods require about 18 hours for determination by manual operation, or 4 to 16 hours even with the introduction of an expensive automatic determination device. Thus, the time required for drug susceptibility test has become an obstacle to selection of the appropriate antimicrobial agent and early starting of treatment.

In an attempt to expedite the testing of drug susceptibility, for example, Patent Literature 2 describes a method for determining drug susceptibility by culturing a bacterium targeted for measurement in two chambers, one containing the antimicrobial agent and the other without it, and measuring their dissolved oxygen concentration to determine the drug susceptibility based on the difference in the activity (oxygen consumption) of the bacterium between two chambers. Moreover, Patent Literature 3 describes a method for determining drug susceptibility based on the changes in the appearance of bacteria given by antimicrobial agents through an observation with an electron microscope, etc. There also is a method utilizing ATP to determine the susceptibility of bacteria to drugs by measuring the amount of ATP derived from dead bacteria in a culture medium containing a drug and bacteria as luminescence utilizing luciferase (Patent Literature 4). However, it takes more than two hours to make a determination even using these techniques, hindering quick selection of antibacterial drugs. Moreover, these techniques require large-scale facilities and special equipment, limiting the institutions that can introduce these testing methods.

In Non-Patent Literatures 4 and 5, systems utilizing NAD(P)H in bacterial cell are reported, which, in principle, utilize the reducing action of NAD(P)H in living bacteria to reduce electron mediators outside the bacterial cell membrane, by adding electron mediators such as menadione to the reaction solution, and colorimetrically measuring the reactive oxygen species produced by the electron mediator-mediated reaction using chemiluminescence methods such as the luminol reaction (Non-Patent Literature 4) or water-soluble formazan dyes (Non-Patent Literature 5). This method requires at least two hours of culturing bacteria in the presence of the antimicrobial agent in order to sufficiently react them before the bacteria solution can be tested, and thus requires at least two hours to make the determination.

It is very important to detect microorganisms in an environment or food for assessing the safety and quality of the environment or food. Various methods are used for such testing, including culturing as a common method. In this method, a sample collected from a food or environment is inoculated into a culture medium, and the presence or absence of colony formation or the turbidity of the liquid medium are visually confirmed. However, this method has to wait for the microorganisms to develop to an extent at which the colonies and the turbidity of the culture medium can be visually confirmed, which might take several days (Patent Literature 5). Moreover, in Patent Literature 5, a method has been developed which combines an image analysis and a deep learning image processing software in detecting microorganisms from images, though the institutions that can introduce these test methods are limited because large-scale facilities and special equipment are indispensable.

As above, the conventional technologies could not actually be said sufficient in promptness of yielding the result and simplicity of testing method both in drug susceptibility testing of microorganisms and in determination of the presence or absence of microorganisms, and could also not be said sufficiently versatile because it was necessary to use large-scale facilities and special equipment.

The present inventors have already established a technique for measuring components such as ammonia in samples using an enzymatic cycling reaction (Patent Literature 6). There have been no reports of performing a drug susceptibility testing or the determination of the presence or absence of microorganisms using such techniques.

Besides, enzymatic cycling reactions are also disclosed in Patent Literatures 7 to 9, etc.

### [Prior Art Literatures]

### [Patent Literatures]

[Patent Literature 1] JP A 2022-113564
[Patent Literature 2] JP A 2020-63959
[Patent Literature 3] JP A 2022-511399
[Patent Literature 4] WO 2016/103433
[Patent Literature 5] JP B 7190534
[Patent Literature 6] JP A 2003-334100
[Patent Literature 7] JP B H02-034599
[Patent Literature 8] JP B H03-053919
[Patent Literature 9] JP B 2579319

### [Non-Patent Literatures]

[Non-Patent Literature 1] Paul M, et al., "Systematic Review and Meta-Analysis of the Efficacy of Appropriate Empiric Antibiotic Therapy for Sepsis", Antimicrob. Agents Chemother., 2010, 54, 4851-4863
[Non-Patent Literature 2] Kumar A, et al., "Duration of hypotension before initiation of effective antimicrobial therapy is the critical determinant of survival in human septic shock", Crit Care Med., 2006, 34, 1589-1596
[Non-Patent Literature 3] Ryuichiro Hori et al., "BISEIBUTUKENSA NO KIHON (Basics of Microbiological Testing) (2) YAKUZAI KANJUSEI SHIKEN (Drug Susceptibility Testing)," The Journal of Japan Society for Clinical Anesthesia, Vol. 37 No. 5, 684-686.
[Non-Patent Literature 4] Shiro Y, et al., "Menadione-Catalyzed O2- Production by Escherichia coli Cells: Application of Rapid Chemiluminescent Assay to Antimicrobial Susceptibility Testing", Microbiol. Immunol., 2001,45(5),333-34
Non-Patent Literature 5] Tadayuki Tsukatani, "Development of a Microbial Detection Method Using the Water-soluble Tetrazolium Salt WST: Food Industry Applications," Nippon Shokuhin Kagaku Kogaku Kaishi (Journal of the Japanese Society for Food Science and Technology) 62 (7), 321-327, 2015

### SUMMARY OF THE INVENTION

### [Problem to be Solved by the Invention]

The object of the present invention is to provide reagents that can be used for drug susceptibility testing of a subject or for determining the presence or absence of the subject and that are versatile and tested by simple methods and are capable of rapidly providing results, which have not been realized by conventional techniques.

### [Means for Solving Problems]

The inventors made a diligent research to solve the problem described above and discovered that it is possible to determine the drug susceptibility or the presence or absence of a cell in a simple and extremely rapid manner by transferring certain intracellular components to the outside the cell and detecting such components using an enzymatic cycling reaction. The inventors further proceeded with the research and reached the completion of the present invention.

Accordingly, the present invention relates to the followings:
[1] A reagent for detecting an intracellular detectable component in a sample, comprising a cell processing agent for transferring the intracellular detectable component to the outside the cell, and a composition for an enzyme cycling reaction for performing the enzyme cycling reaction to the detectable component.
[2] The reagent according to [1], wherein the cell is a bacterial or fungal microbial cell.
[3] The reagent according to [2], for determining the susceptibility of the microbial cell to the cell processing agent or for determining the presence or absence of the microbial cell in the sample.
[4] The reagent according to any one of [1] to [3], wherein the cell processing agent is one or more selected from the group consisting of antimicrobial agents, benzalkonium chloride, cetyltrimethylammonium bromide, and cetyltrimethylammonium chloride.
[5] The reagent according to [4], wherein the antibacterial agent is one or more selected from the group consisting of cephem antimicrobial agents, penem antimicrobial agents, quinolone antimicrobial agents, penicillin antimicrobial agents, glycopeptide antimicrobial agents, and aminoglycoside antimicrobial agents.
[6] The reagent according to any one of [1] to [5], wherein the detectable component is one or more selected from the group consisting of NAD+, NADP+, NADH and NADPH.
[7] The reagent according to any one of [1] to [6], wherein the composition for enzymatic cycling reaction comprises glucose, glucose dehydrogenase, a substrate of diaphorase and diaphorase.
[8] The reagent according to [7], wherein the substrate of diaphorase is a tetrazolium salt.
[9] The reagent according to [8], wherein the tetrazolium salt is WST-8, 3-(4,5-dimethyl-2-thiazolyl)-2,5- diphenyltetrazolium bromide or Tetranitro Blue Tetrazolium.
[10] The reagent according to any one of [1] to [9], further comprising a nonionic surfactant.
[11] The reagent according to any one of [2] to [10], for determining the susceptibility of a microbial cell to a cell processing agent, further comprising cell culture medium ingredients that either do not contain the detectable component or contains it only at a concentration of 5 µg/L or less.
[12] The reagent according to [11], wherein the cell culture medium ingredients are peptone and chloride.
[13] A method for detecting an intracellular detectable component in a sample, comprising detecting the detectable component that has been transferred to the outside of the cell by a cell processing agent using an enzymatic cycling reaction within the reagent according to any one of [1] to [12].
[14] The method according to [13], wherein the cell is a bacterial, fungal or yeast microbial cell, and the method is for determining the susceptibility of the microbial cell to the cell processing agent or for determining the presence or absence of the microbial cell in the sample.
[15] A method for detecting an intracellular detectable component in a sample, wherein the method is for determining the susceptibility of microbial cell to a cell processing agent, and wherein the method comprises detecting the detectable component that has been transferred to the outside the cell by the cell processing agent using an enzyme cycling reaction within the reagent according to [11] or [12].

### [Advantageous Effects of the Invention]

By using the reagents of the present invention, it is possible to detect simply and quickly that cells such as bacteria or fungi have been destroyed or lysed by a cell processing agent. By using the reagents of the present invention, it is possible to determine the drug susceptibility of microorganisms such as bacteria or the presence or absence of microorganisms, in an extremely short time such as about one hour and in a simple manner, which were both not possible with conventional techniques. It is also highly versatile, requiring no large-scale facilities or special equipment.

The reagent of the present invention can be used, when the cell processing agent is a certain antimicrobial agent or the like, in a testing method for determining whether a microorganism to be tested, such as a bacterium or fungus, is susceptible to the antimicrobial agent. The reagent of the present invention can be used, when the cell processing agent is a bactericide or like, in a testing method for investigating whether a microorganism to be tested, such as a bacterium or fungus, is present.

For drug susceptibility testing, it takes 18 hours for determination using conventional methods such as micro-liquid dilution and disk diffusion methods by manual operation, or 4 to 16 hours even with the introduction of an expensive automatic determination machinery. On the other hand, using the reagent of the present invention, the drug susceptibility can be determined in about one hour. Moreover, once the reaction solution has been inoculated with the specimen such as bacteria and incubated thereafter, the reagent of the present invention enables visual determination of the presence or absence of coloration of the reaction solution, and thus does not require any complicated or special operations, nor does it require any large-scale equipment or special implements.

The reagent of the present invention is expected to contribute to quick selection of antimicrobial agents, since it is capable of determining whether the target bacteria is susceptible to a drug in about one hour by simple operation, without using any large-scale equipment or special implements.

For detection of microorganisms, general culture methods require 1 to 3 days of culturing time before colonies that are large enough to be visible are formed. There also is a fluorescence detection method in which a liquid sample, etc., is filtered through a membrane filter, and this membrane filter is affixed to a plate medium and cultured, and the resulting minute microcolonies are fluorescently stained for conducting fluorescence observation. Even introducing such expensive equipment, it takes 3 to 24 hours. On the other hand, using the reagent of the present invention, the presence or absence of microorganisms can be determined in about one hour. In addition, the reagent of the present invention does not require any specialized operations or special equipment because it enables visual determination of changes in color tone of the reaction solution after the specimen is inoculated into the reaction solution.

In conventional detection methods using the luminol reaction, for example, luminescence by luminol lasts only for several tens of seconds. Therefore, if a cell processing agent is added to the reagent in advance, the luminescence would be over before the timing at which the drug susceptibility can be determined, making the determination (detection of luminescence) impossible. In contrast, in the present invention, the dye (color tone) produced by the enzymatic cycling reaction would accumulate rather than being attenuated, allowing the cell processing agent to be included in the reagent in advance, making the detection possible with a simple operation without a need for adding any additional reagents at the time of detection.

Since the reagent of the present invention can determine the presence or absence of microorganisms with a sample operation and in a time as short as one hour without using any specialized equipment, it is expected to contribute, for example, to shortening the post-production time before shipment at soft drink factories and to hygiene control at small-scale factories.

### BRIEF DESCRIPTION OF DRAWINGS

[FIG. 1] FIG. 1 is a explanatory diagram illustrating the principle of detection by the present invention.
[FIG. 2] FIG. 2 is a diagram showing the coloration presented by susceptible and resistant bacteria in the product of the present invention using WST-8 as the substrate. It will turns yellow with a susceptible bacterium and remains clear and colorless with a resistant bacterium.

### DESCRIPTION OF EMBODIMENTS

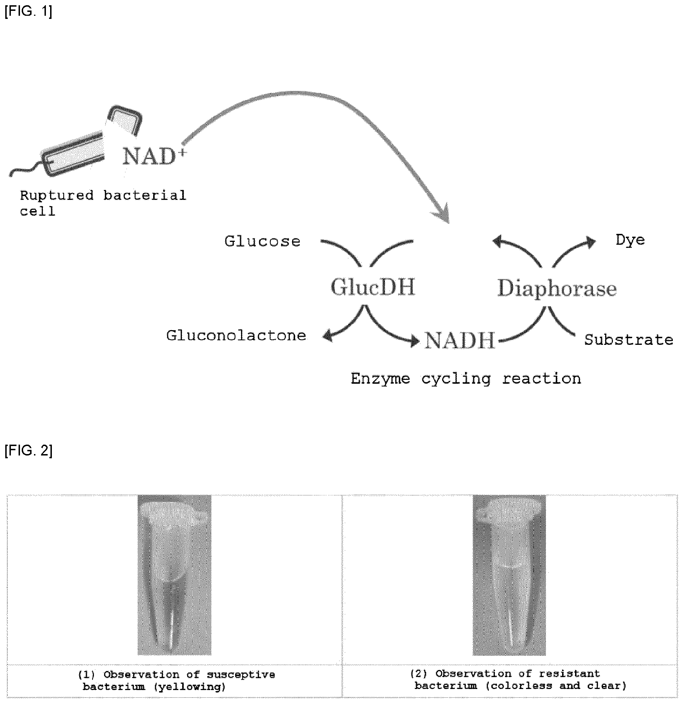

The principle of detection using the reagents of the present invention will be explained as follows using FIG. 1 using bacteria as an example.

In a living bacterial cell, there exist a coenzyme (NAD+), which are essential for vital activities. When an antimicrobial agent is applied to a certain bacterium, the antimicrobial agent is able to rupture the bacterial cell and kill it if it is effective against that bacterium. The NAD+ released from this ruptured bacterial cell is converted to a detectable formazan dye by utilizing an enzymatic cycling reaction (FIG. 1).

In detail, the released NAD+ is converted to NADH by glucose and glucose dehydrogenase (hereinbelow "GlucDH"). The NADH becomes NAD+ again in the presence of a substrate such as a tetrazolium salt and diaphorase while giving rise to a formazan dye. Furthermore, the NAD+ becomes NADH again by the enzymatic reaction of GlucDH, and the reaction is repeated such that the NADH again returns to NAD+ by the enzymatic reaction of diaphorase, resulting in the accumulation of the formazan dye which causes a change in the color of the reaction solution. Thus, the bacterium can be determined to be susceptible to the antimicrobial agent (effective) if the color of the reaction solution changes, and the bacterium can be determined to be resistant to the antimicrobial agent (ineffective) if the color of the medium does not change.

Moreover, a bactericide which destroys the cell membranes of most microorganisms (such as benzalkonium chloride) can be used in place of an antimicrobial agent as a reagent to detect microorganisms. Specifically, if microorganisms are present in the target sample, the microorganisms are ruptured by the bactericide, and the released NAD+ gives rise to a formazan dye as described above, which would change the color of the reaction solution. Therefore, if the color of the reaction solution changes, it can be determined that microorganisms are present in the sample, and if the color of the reaction solution does not change, it can be determined that no microorganisms are present.

The present invention in one embodiment relates to a reagent for detecting an intracellular detectable component in a sample, comprising a cell processing agent for transferring the intracellular detectable component to the outside the cell, and a composition for an enzyme cycling reaction for performing the enzyme cycling reaction to the detectable component. The reagent of the present invention does not contain the detectable component or contains it only at a concentration of 5 µg/L or less. Even if the detectable component is contained, the enzymatic cycling reaction for one hour does not cause any change in color tone at this level of concentration. That is, it does not show false positive results, and thus does not affect the detection results.

Here, an enzymatic cycling reaction refers, to give an example, to a repetitive reaction in which, when NAD+ is supplied, a first enzyme catalyzes a reaction that consumes NAD+ and a first substrate to produce NADH and an oxidation product of the first substrate, and a second enzyme catalyzes a reaction that consumes the NADH and a second substrate to produce NAD+ and a reduction product of the second substrate.

An example of an enzymatic cycling reaction is shown in FIG. 1. In FIG. 1, NAD+ is supplied and an enzymatic cycling reaction takes place. The first substrate is glucose, the oxidation product of the first substrate is gluconolactone, and the first enzyme is glucose dehydrogenase (GlucDH) (FIG. 1, left side). The second substrate is a substrate such as tetrazolium salt, the reduction product of the second substrate is a dye such as a formazan dye, and the second enzyme is a diaphorase (FIG. 1, right side).

The cells in the present invention are not particularly limited as long as they are capable of utilizing the principle of detection using an enzymatic cycling reaction, and can be animal cells, plant cells, or microbial cells such as bacteria or fungi. The reagent of the present invention is capable of determining the drug susceptibility and presence or absence of these cells in a sample. The cells are preferably microbial cells such as bacterial or fungal cells.

Bacteria include *Escherichia coli, Staphylococcus aureus, Klebsiella pneumoniae, Klebsiella oxytoca, Serratia marcescens, Proteus mirabilis, Proteus vulgaris, Providencia rettgeri, Morganella morganii, Citrobacter freundii, Citrobacter koseri, Enterobacter cloacae, Enterobacter aerogenes, Enterobacter asburiae, Pseudomonas aeruginosa, Pseudomonas putida, Stenotrophomonas multophilia, Acinetobacter baumannii, Aeromonas hydrophila, Moraxella catarrhalis, Hafnia alvei, Sphingomonas paucimobilis, Streptococcus pneumoniae, Streptococcus pyogenes, Staphylococcus epidermidis, Enterococcus fecalis, Enterococcus feacium, Enterococcus casseliflavus,* and the like.

Fungi include *Aspergillosis fumigatus, Aspergillosis flavus, Aspergillosis niger, Candida auris, Candida albicans, Candida tropicalis, Candida krusei, Candida glabrata, Cryptococcus neoformans,* and the like.

The cell processing agent of the present invention is not particularly limited as long as it is a drug that is capable of destroying or lysing the target cell. It can appropriately be selected according to the type of the cell. For instance, when the target cell is a bacterial cell, antimicrobial agents (cephem antimicrobial agents, penem antimicrobial agents, quinolone antimicrobial agents, penicillin antimicrobial agents, aminoglycoside antimicrobial agents, etc.), benzalkonium chloride, cetyltrimethylammonium bromide, cetyltrimethylammonium chloride, and the like, are used.

Cephem antimicrobial agents include cefepime, ceftazidime, cefpodoxime, cefotaxime, cefazolin, cephalexin, and the like.

Penem antimicrobial agents include imipenem, meropenem, ertapenem, faropenem, and the like.

Quinolone antimicrobial agents include ciprofloxacin (CPFX), levofloxacin, ofloxacin, norofloxacin, and the like.

Penicillin antimicrobial agents include ampicillin, cloxacillin, and the like.

Glycopeptide antimicrobial agents include vancomycin and the like.

Aminoglycoside antimicrobial agents include amikacin, tobramycin, arbekacin, kanamycin, and the like.

Detectable components in the present invention include NAD+, NADP+, NADH, NADPH, and the like. As detectable components, NAD+, NADP+, NADH, and NADPH are preferred from the viewpoint that the product after the enzymatic cycling reaction is a water soluble formazan that can be visually observed as a dye.

In the present invention, a composition for an enzymatic cycling reaction refers to a composition comprising the components that are necessary for the enzymatic cycling reaction. The components that are necessary for the enzymatic cycling reaction include the first substrate, the first enzyme, the second substrate, and the second enzyme, as described above.

The combination of the substrate and the enzyme can appropriately be selected by a person skilled in the art according to the detectable component (the substance supplied to the enzyme cycling reaction system), based on descriptions in prior art literatures such as Patent literatures 6 to 9, etc.

For instance, when the detectable component is NAD+, NADP+, NADH, or NADPH, the first substrate, the oxidation product of the first substrate, and the first enzyme of the enzyme cycling reaction can be glucose, glucono-δ-lactone, and glucose dehydrogenase, respectively, while the second substrate, the oxidation product of the second substrate, and the second enzyme can be WST-8, soluble formazan, an diaphorase, respectively.

In one embodiment, the composition for an enzymatic cycling reaction comprises glucose, glucose dehydrogenase, a substrate for diaphorase, and diaphorase.

The substrate for diaphorase may be a tetrazolium salt, where the tetrazolium salt is preferably WST-8, 3-(4,5-dimethyl-2-thiazolyl)-2,5-diphenyltetrazolium bromide or Tetranitro Blue Tetrazolium.

The reagent of the present invention may, in one embodiment, further comprise a nonionic surfactant. Nonionic surfactants include, for example, polyethylene glycol sorbitan monolaurate (Tween 20), polyoxyethylene sorbitan monopalmitate (Tween 40), polyethylene glycol sorbitan monostearate (Tween 60), oleic acid polyoxyethylene sorbitan (Tween 80), polyethylene glycol tert-octyl phenyl ether (Triton^{™} X-405), and the like. It has been known that the inclusion of a nonionic surfactant has an effect on the permeability of microbial cell membrane to increase the efficiency of nutrient component uptake. Furthermore, it improves the dispersibility of microorganisms in the reagent, allowing the growth of the microorganisms to be promoted. This allows an antimicrobial agent such as a cephem, quinolone or aminoglycoside antimicrobial agent, which has an effect of rupturing microorganisms as they divide, to act effectively against the microorganisms.

The reagent of the present invention may, in one embodiment, further comprise a cell culture medium ingredient to determine the susceptibility of the bacteria to the cell processing agent. Here, the cell culture medium ingredient does not contain the detectable component or contains it only at a concentration of 5 µg/L or less at which no change in color tune would occur in a one-hour enzyme cycling reaction. For instance, when the detectable component in the cell is NAD+, the cell culture medium ingredient either contains no NAD+ or contains it only at a concentration of 5 µg/L or less at which no change in color tune would occur in a one-hour enzyme cycling reaction. The inclusion of such a medium component promotes cell growth (including processes such as cell division), allows the cell processing agent to act efficiently, and makes it easy to determine the susceptibility of bacteria to the cell processing agent. The cell culture medium ingredient that can be used in the present invention is not limited as long as it does not contain the detectable component or contains it only at a concentration of 5 µg/L or less at which no change in color tune would occur in a one-hour enzyme cycling reaction, provided that it is an ingredient that is generally known as a culture medium ingredient for that cell. For instance, when the detectable component is NAD+, tryptone, Bacto^{™} peptone, glucose, xylose, sodium chloride, potassium chloride and the like can be used as the cell culture medium ingredients.

The cell culture medium ingredients are preferably peptone and chloride. Here, peptones include tryptone (casein peptone), Bacto^{™} peptone (meat peptone), heart peptone (cardiac muscle peptone), soy peptone, gelatin peptone, and the like. In the reagent of the present invention, peptone is added as a nitrogen source (amino acid source) for the microorganisms, and without this, the microorganisms cannot divide (grow). For instance, cephem antimicrobial agents inhibit synthesis of cell wall by binding to cell wall synthesizing enzymes. If the microorganism tries to divide while cell wall synthesis is inhibited, the cell wall becomes so thin that it cannot withstand the osmotic pressure difference with the outer fluid and causes the cell to rupture . However, in the absence of a nitrogen source, the microorganism does not attempt to divide (multiply), causing no rupture of the cell. Therefore, in the absence of a nitrogen source, even if the microorganism is susceptible to cephem antimicrobial agents, its cell will not rupture and thus will be incorrectly determined to be resistant. This false determination can be prevented by including peptone in the reagent of the present invention.

On the other hand, chlorides refers to, for example, potassium chloride, sodium chloride, and the like, and, by including these, the osmotic pressure inside and outside the body of a microorganism can be adjusted. During division, microorganisms undergo cytoplasmic expansion and cell wall synthesis. There are cases, however, where this balance is disrupted and cell division occurs with incomplete cell wall synthesis. At that time, in a hypotonic solution with no chloride in the reagent, the osmotic pressure causes the cell to rupture. Therefore, for example, even if a microorganism is resistant to cephem antimicrobial agents, osmotic pressure will cause its cell to rupture, and the cell will be incorrectly determined to be susceptible. This false determination can be prevented by including chloride in the reagent.

The present invention also relates to a method for detecting an intracellular detectable component in a sample, the method comprising detecting the detectable component that has been transferred to the outside of the cell by a cell processing agent using an enzymatic cycling reaction within the reagent of the present invention.

The present invention may, in one embodiment, where the cell is a bacterial cell, be a method for determining the susceptibility of the bacterium to a cell processing agent or for determining the presence or absence of the bacterium in the sample. Determination can be confirmed by visual inspection or by measuring the absorbance of the dye produced by the enzymatic cycling reaction.

The present invention, in one embodiment, relates to a method for detecting an intracellular detectable component in a sample, wherein the method is for determining the susceptibility of bacteria to a bactericidal ingredient, and wherein the method comprises detecting the detectable component that has been transferred to the outside the cell by a cell processing agent using an enzyme cycling reaction within the reagent of the present invention comprising peptone and chloride.

### [Examples]

### [Experiment 1] Test for drug susceptibility of bacteria

### 1. Preparation of the reagent of the present invention

The composition of the reagent of the present invention (hereinbelow referred to as "Reagent 1") that was provided for testing is as follows.

### <Composition of Reagent 1>

| | |
|---|---|
| Sodium dihydrogen phosphate, dihydrate | 3.9 g/L |
| Disodium hydrogen phosphate, 12-hydrate | 2.5 g/L |
| Sodium chloride | 1.0 g/L |
| Potassium chloride | 6.0 g/L |
| Tween 20 | 1.0 g/L |
| Tryptone | 0.5 g/L |
| Bacto^{™} peptone | 2.0 g/L |
| Xylose | 1.0 g/L |

### (1) Cell processing agents:

Drugs described in Tables 1, 3, 5, 7, 9, 11, 13, and 15 (2) Composition for enzymatic cycling reaction:

| | |
|---|---|
| Glucose | 5.0 g/L |
| Glucose dehydrogenase | 3750 U/L |
| WST-8 | 270 mg/L |
| Diaphorase | 1 KU/L |

Note that WST-8 is a tetrazolium salt that is a substrate for diaphorase and produces a yellow water-soluble formazan by enzymatic cycling reaction.

### 2. Bacteria provided for testing

Determination of the drug susceptibility using Reagent 1 was carried out using bacteria indicated in Table 1 (20 species, 234 strains), and compared to another drug susceptibility determination by the disk diffusion method performed using KB-disc^{®} (Eiken Chemical Co., Ltd.) cefpodoxime according to the package insert of this reagent.

**[Table 1]**

| | Bacterium (scientific name) | Number of used strains | Drug (cell processing agent) |
|---|---|---|---|
| 1 | Citrobacter amalonaticus | 1 strain | 100 mg/L cefpodoxime |
| 2 | Citrobacter diversus | 1 strain | |
| 3 | Citrobacter freundii | 9 strains | |
| 4 | Enterobacter aerogenes | 5 strains | |
| 5 | Enterobacter asburiae | 2 strains | |
| 6 | Enterobacter amnigenus | 2 strains | |
| 7 | Enterobacter cloacae | 23 strains | |
| 8 | Escherichia albertii | 1 strain | |
| 9 | Escherichia coli | 86 strains | |
| 10 | Escherichia hermannii | 2 strains | |
| 11 | Escherichia fergusonii | 1 strain | |
| 12 | Hafnia alvei | 2 strains | |
| 13 | Klebsiella oxytoca | 15 strains | |
| 14 | Klebsiella pneumoniae | 65 strains | |
| 15 | Kluyvera citrophila | 1 strain | |
| 16 | Morganella morganii | 4 strains | |
| 17 | Proteus mirabilis | 7 strains | |
| 18 | Proteus vulgaris | 3 strains | |
| 19 | Providencia rettgeri | 1 strain | |
| 20 | Serratia marcescens | 5 strains | |

Isolated bacteria (20 species, 234 strains) were each smeared on CHROMagar^{™} Orientation agar medium, and cultured at 37 °C for 24 hours. Then, the colonies that developed on the medium were picked up and suspended in sterile physiological saline to be approximately McFarland turbidity 2, which was used as the bacterial suspension.

### 3. Determination of drug susceptibility using Reagent 1

One hundred microliter of each bacterial suspension prepared in 2 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 4. Determination of drug susceptibility using disk diffusion method

Using the bacterial suspension of each bacterium prepared in 2 above, the drug susceptibility for each bacterium was determined according to the disk diffusion method performed using KB-disc^{®} (Eiken Chemical Co., Ltd.) cefpodoxime according to the package insert of this reagent (cultured at 37 °C for 18 hours).

### 5. Results

The results are shown in Table 2.

**[Table 2]**

| | | Disk diffusion method | |
|---|---|---|---|
| | | Susceptibility | Resistance |
| The method of the present invention | Susceptibility | 86 (89%) | 11 |
| | Resistance | 11 | 126 (92%) |

| | | | |
|---|---|---|---|
| *Numbers in parentheses indicate agreement rates. Number of tested bacterial strains: 234 strains. | | | |

The results of the determination of the method using Reagent 1 were compared with those of the disk diffusion method (Table 2), and there was 89% agreement for bacteria determined to be susceptible and 92% agreement for bacteria determined to be resistant. These results indicate that the method using Reagent 1 can give a result of drug susceptibility determination that has a high agreement rate with the conventional method in a very short time.

### 6. Bacteria provided for testing

Determination of the drug susceptibility using Reagent 1 was carried out using bacteria indicated in Table 3 (11 species, 94 strains), and compared to another drug susceptibility determination by the disk diffusion method performed using KB-disc^{®} (Eiken Chemical Co., Ltd.) cefotaxime according to the package insert of this reagent.

**[Table 3]**

| | Bacterium (scientific name) | Number of used strains | Drug (cell processing agent) |
|---|---|---|---|
| 1 | Citrobacter freundii | 5 strains | |
| 2 | Enterobacter aerogenes | 5 strains | |
| 3 | Enterobacter asburiae | 2 strains | |
| 4 | Enterobacter cloacae | 10 strains | |
| 5 | Escherichia coli | 40 strains | |
| 6 | Klebsiella oxytoca | 5 strains | 80 mg/L cefotaxime |
| 7 | Klebsiella pneumoniae | 20 strains | |
| 8 | Proteus mirabilis | 3 strains | |
| 9 | Proteus vulgaris | 3 strains | |
| 10 | Serratia marcescens | 2 strains | |
| 11 | Stenotrophomonas maltophilia | 2 strains | |

Isolated bacteria (11 species, 94 strains) were each smeared on blood agar medium, and cultured at 37 °C for 24 hours. Then, the colonies that developed on the medium were picked up and suspended in sterile physiological saline to be approximately McFarland turbidity 2, which was used as the bacterial suspension.

### 7. Determination of drug susceptibility using Reagent 1

One hundred microliter of each bacterial suspension prepared in 6 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 8. Determination of drug susceptibility using disk diffusion method

Using the bacterial suspension of each bacterium prepared in 6 above, the drug susceptibility for each bacterium was determined according to the disk diffusion method performed using KB-disc^{®} (Eiken Chemical Co., Ltd.) cefotaxime according to the package insert of this reagent (cultured at 37 °C for 18 hours).

### 9. Results

The results are shown in Table 4.

**[Table 4]**

| | | Disk diffusion method | |
|---|---|---|---|
| | | Susceptibility | Resistance |
| The method of the present invention | Susceptibility | 51 (97%) | 6 |
| | Resistance | 2 | 35 (85%) |

| | | | |
|---|---|---|---|
| *Numbers in parentheses indicate agreement rates. Number of tested bacterial strains: 94 strains. | | | |

The results of the determination of the method using Reagent 1 were compared with those of the disk diffusion method (Table 4), and there was 97% agreement for bacteria determined to be susceptible and 85% agreement for bacteria determined to be resistant. These results indicate that the method using Reagent 1 can give a result of drug susceptibility determination that has a high agreement rate with the conventional method in a very short time.

### 10. Bacteria provided for testing

Determination of the drug susceptibility using Reagent 1 was carried out using bacteria indicated in Table 5 (11 species, 94 strains), and compared to another drug susceptibility determination by the disk diffusion method performed using KB-disc^{®} (Eiken Chemical Co., Ltd.) ceftazidime according to the package insert of this reagent.

**[Table 5]**

| | Bacterium (scientific name) | Number of used strains | Drug (cell processing agent) |
|---|---|---|---|
| 1 | Citrobacter freundii | 5 strains | |
| 2 | Enterobacter aerogenes | 5 strains | |
| 3 | Enterobacter asburiae | 2 strains | |
| 4 | Enterobacter cloacae | 10 strains | |
| 5 | Escherichia coli | 40 strains | |
| 6 | Klebsiella oxytoca | 5 strains | 80 mg/L ceftazidime |
| 7 | Klebsiella pneumoniae | 20 strains | |
| 8 | Proteus mirabilis | 3 strains | |
| 9 | Proteus vulgaris | 3 strains | |
| 10 | Serratia marcescens | 2 strains | |
| 11 | Stenotrophomonas maltophilia | 2 strains | |

Isolated bacteria (11 species, 94 strains) were each smeared on CHROMagar^{™} Orientation agar medium, and cultured at 37 °C for 24 hours. Then, the colonies that developed on the medium were picked up and suspended in sterile physiological saline to be approximately McFarland turbidity 2, which was used as the bacterial suspension.

### 11. Determination of drug susceptibility using Reagent 1

One hundred microliter of each bacterial suspension prepared in 10 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 12. Determination of drug susceptibility using disk diffusion method

Using the bacterial suspension of each bacterium prepared in 10 above, the drug susceptibility for each bacterium was determined according to the disk diffusion method performed using KB-disc^{®} (Eiken Chemical Co., Ltd.) ceftazidime according to the package insert of this reagent (cultured at 37 °C for 18 hours).

### 13. Results

The results are shown in Table 6.

**[Table 6]**

| | | Disk diffusion method | |
|---|---|---|---|
| | | Susceptibility | Resistance |
| The method of the present invention | Susceptibility | 45 (88%) | 2 |
| | Resistance | 6 | 41 (95%) |

| | | | |
|---|---|---|---|
| *Numbers in parentheses indicate agreement rates. Number of tested bacterial strains: 94 strains. | | | |

The results of the determination of the method using Reagent 1 were compared with those of the disk diffusion method (Table 6), and there was 88% agreement for bacteria determined to be susceptible and 95% agreement for bacteria determined to be resistant. These results indicate that the method using Reagent 1 can give a result of drug susceptibility determination that has a high agreement rate with the conventional method in a very short time.

### 14. Bacteria provided for testing

Determination of the drug susceptibility using Reagent 1 was carried out using bacterium indicated in Table 7 (5 species, 8 strains), and compared to another drug susceptibility determination by the disk diffusion method performed using KB-disc^{®} (Eiken Chemical Co., Ltd.) levofloxacin according to the package insert of this reagent.

**[Table 7]**

| | Bacterium (scientific name) | Number of used strains | Drug (cell processing agent) |
|---|---|---|---|
| 1 | Citrobacter freundii | 1 strain | 8 mg/L levofloxacin |
| 2 | Enterobacter aerogenes | 1 strain | |
| 3 | Enterobacter cloacae | 2 strains | |
| 4 | Escherichia coli | 2 strains | |
| 5 | Klebsiella pneumoniae | 2 strains | |

Isolated bacteria (5 species, 8 strains) were each smeared on CHROMagar^{™} Orientation agar medium, and cultured at 37 °C for 24 hours. Then, the colonies that developed on the medium were picked up and suspended in sterile physiological saline to be approximately McFarland turbidity 2, which was used as the bacterial suspension.

### 15. Determination of drug susceptibility using Reagent 1

One hundred microliter of each bacterial suspension prepared in 14 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 16. Determination of drug susceptibility using disk diffusion method

Using the bacterial suspension of each bacterium prepared in 14 above, the drug susceptibility for each bacterium was determined according to the disk diffusion method performed using KB-disc^{®} (Eiken Chemical Co., Ltd.) ceftazidime according to the package insert of this reagent (cultured at 37 °C for 18 hours).

### 17. Results

The results are shown in Table 8.

**[Table 8]**

| | | Disk diffusion method | |
|---|---|---|---|
| | | Susceptibility | Resistance |
| The method of the present invention | Susceptibility | 3 (75%) | 0 |
| | Resistance | 1 | 4 (100%) |

| | | | |
|---|---|---|---|
| *Numbers in parentheses indicate agreement rates. Number of tested bacterial strains: 8 strains. | | | |

The results of the determination of the method using Reagent 1 were compared with those of the disk diffusion method (Table 8), and there was 75% agreement for bacteria determined to be susceptible and 100% agreement for bacteria determined to be resistant. These results indicate that the method using Reagent 1 can give a result of drug susceptibility determination that has a high agreement rate with the conventional method in a very short time.

### 18. Bacteria provided for testing

Determination of the drug susceptibility using Reagent 1 was carried out using bacteria indicated in Table 9 (5 species, 8 strains), and compared to another drug susceptibility determination by the disk diffusion method performed using KB-disc^{®} (Eiken Chemical Co., Ltd.) amikacin according to the package insert of this reagent.

**[Table 9]**

| | Bacterium (scientific name) | Number of used strains | Drug (cell processing agent) |
|---|---|---|---|
| 1 | Citrobacter freundii | 1 strain | 1 g/L amikacin |
| 2 | Enterobacter aerogenes | 1 strain | |
| 3 | Enterobacter cloacae | 2 strains | |
| 4 | Escherichia coli | 2 strains | |
| 5 | Klebsiella pneumoniae | 2 strains | |

Isolated bacteria (5 species, 8 strains) were each smeared on CHROMagar^{™} Orientation agar medium, and cultured at 37 °C for 24 hours. Then, the colonies that developed on the medium were picked up and suspended in sterile physiological saline to be approximately McFarland turbidity 2, which was used as the bacterial suspension.

### 19. Determination of drug susceptibility using Reagent 1

One hundred microliter of each bacterial suspension prepared in 18 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 20. Determination of drug susceptibility using disk diffusion method

Using the bacterial suspension of each bacterium prepared in 18 above, the drug susceptibility for each bacterium was determined according to the disk diffusion method performed using KB-disc^{®} (Eiken Chemical Co., Ltd.) amikacin according to the package insert of this reagent (cultured at 37 °C for 18 hours).

### 21. Results

The results are shown in Table 10.

**[Table 10]**

| | | Disk diffusion method | |
|---|---|---|---|
| | | Susceptibility | Resistance |
| The method of the present invention | Susceptibility | 2 (50%) | 0 |
| | Resistance | 2 | 4 (100%) |

| | | | |
|---|---|---|---|
| *Numbers in parentheses indicate agreement rates. Number of tested bacterial strains: 8 strains. | | | |

The results of the determination of the method using Reagent 1 were compared with those of the disk diffusion method (Table 10), and there was 50% agreement for bacteria determined to be susceptible and 100% agreement for bacteria determined to be resistant. These results indicate that the method using Reagent 1 can give a result of drug susceptibility determination that has a high agreement rate with the conventional method in a very short time.

### 22. Bacteria provided for testing

Determination of the drug susceptibility using Reagent 1 was carried out using bacteria indicated in Table 11 (5 species, 8 strains), and compared to another drug susceptibility determination by the disk diffusion method performed using KB-disc^{®} (Eiken Chemical Co., Ltd.) meropenem according to the package insert of this reagent.

**[Table 11]**

| | Bacterium (scientific name) | Number of used strains | Drug (cell processing agent) |
|---|---|---|---|
| 1 | Citrobacter freundii | 1 strain | 2 mg/L meropenem |
| 2 | Enterobacter aerogenes | 1 strain | |
| 3 | Enterobacter cloacae | 2 strains | |
| 4 | Escherichia coli | 2 strains | |
| 5 | Klebsiella pneumoniae | 2 strains | |

Isolated bacteria (5 species, 8 strains) were each smeared on CHROMagar^{™} Orientation agar medium, and cultured at 37 °C for 24 hours. Then, the colonies that developed on the medium were picked up and suspended in sterile physiological saline to be approximately McFarland turbidity 2, which was used as the bacterial suspension.

### 23. Determination of drug susceptibility using Reagent 1

One hundred microliter of each bacterial suspension prepared in 22 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 24. Determination of drug susceptibility using disk diffusion method

Using the bacterial suspension of each bacterium prepared in 22 above, the drug susceptibility for each bacterium was determined according to the disk diffusion method performed using KB-disc^{®} (Eiken Chemical Co., Ltd.) meropenem according to the package insert of this reagent (cultured at 37 °C for 18 hours).

### 25. Results

The results are shown in Table 12.

**[Table 12]**

| | | Disk diffusion method | |
|---|---|---|---|
| | | Susceptibility | Resistance |
| The method of the present invention | Susceptibility | 3 (75%) | 0 |
| | Resistance | 1 | 4 (100%) |

| | | | |
|---|---|---|---|
| *Numbers in parentheses indicate agreement rates. Number of tested bacterial strains: 8 strains. | | | |

The results of the determination of the method using Reagent 1 were compared with those of the disk diffusion method (Table 12), and there was 75% agreement for bacteria determined to be susceptible and 100% agreement for bacteria determined to be resistant. These results indicate that the method using Reagent 1 can give a result of drug susceptibility determination that has a high agreement rate with the conventional method in a very short time.

### 26. Bacteria provided for testing

Determination of the drug susceptibility using Reagent 1 was carried out using bacteria indicated in Table 13 (2 species, 6 strains), and compared to another drug susceptibility determination by the disk diffusion method performed using KB-disc^{®} (Eiken Chemical Co., Ltd.) ampicillin according to the package insert of this reagent.

**[Table 13]**

| | Bacterium (scientific name) | Number of used strains | Drug (cell processing agent) |
|---|---|---|---|
| 1 | Staphylococcus aureus | 3 strain | 2 mg/L ampicillin |
| 2 | Staphylococcus epidermidis | 3 strain | |

Isolated bacteria (2 species, 6 strains) were each smeared on Soybean-Casein-Digest agar medium, and cultured at 37 °C for 24 hours. Then, the colonies that developed on the medium were picked up and suspended in sterile physiological saline to be approximately McFarland turbidity 2, which was used as the bacterial suspension.

### 27. Determination of drug susceptibility using Reagent 1

One hundred microliter of each bacterial suspension prepared in 26 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 28. Determination of drug susceptibility using disk diffusion method

Using the bacterial suspension of each bacterium prepared in 26 above, the drug susceptibility for each bacterium was determined according to the disk diffusion method performed using KB-disc^{®} (Eiken Chemical Co., Ltd.) ampicillin according to the package insert of this reagent (cultured at 37 °C for 18 hours).

### 29. Results

The results are shown in Table 14.

**[Table 14]**

| | | Disk diffusion method | |
|---|---|---|---|
| | | Susceptibility | Resistance |
| The method of the present invention | Susceptibility | 3 (100%) | 0 |
| | Resistance | 0 | 3 (100%) |

| | | | |
|---|---|---|---|
| *Numbers in parentheses indicate agreement rates. Number of tested bacterial strains: 6 strains. | | | |

The results of the determination of the method using Reagent 1 were compared with those of the disk diffusion method (Table 14), and there was 100% agreement for bacteria determined to be susceptible and 100% agreement for bacteria determined to be resistant. These results indicate that the method using Reagent 1 can give a result of drug susceptibility determination that has a high agreement rate with the conventional method in a very short time.

### 30. Bacteria provided for testing

Determination of the drug susceptibility using Reagent 1 was carried out using bacteria indicated in Table 15 (4 species, 8 strains), and compared to another drug susceptibility determination by the disk diffusion method performed using KB-disc^{®} (Eiken Chemical Co., Ltd.) vancomycin according to the package insert of this reagent.

**[Table 15]**

| | Bacterium (scientific name) | Number of used strains | Drug (cell processing agent) |
|---|---|---|---|
| 1 | Staphylococcus aureus | 2 strains | 32 mg/L vancomycin |
| 2 | Staphylococcus epidermidis | 2 strains | |
| 3 | Enterococcus faecalis | 2 strains | |
| 4 | Enterococcus faecium | 2 strains | |

Isolated bacteria (4 species, 8 strains) were each smeared on Soybean-Casein-Digest agar medium, and cultured at 37 °C for 24 hours. Then, the colonies that developed on the medium were picked up and suspended in sterile physiological saline to be approximately McFarland turbidity 2, which was used as the bacterial suspension.

### 31. Determination of drug susceptibility using Reagent 1

One hundred microliter of each bacterial suspension prepared in 30 above was inoculated to Reagent 1 (1 mL), incubated at 37 °C for 1 hour, and then the drug susceptibility of each bacterium was determined. Determination was made based on whether or not Reagent 1 turned yellow by visual inspection.

### 32. Determination of drug susceptibility using disk diffusion method

Using the bacterial suspension of each bacterium prepared in 30 above, the drug susceptibility for each bacterium was determined according to the disk diffusion method performed using KB-disc^{®} (Eiken Chemical Co., Ltd.) vancomycin according to the package insert of this reagent (cultured at 37 °C for 18 hours).

### 33. Results

The results are shown in Table 16.

**[Table 16]**

| | | Disk diffusion method | |
|---|---|---|---|
| | | Susceptibility | Resistance |
| The method of the present invention | Susceptibility | 3 (100%) | 1 |
| | Resistance | 0 | 4 (80%) |

| | | | |
|---|---|---|---|
| *Numbers in parentheses indicate agreement rates. Number of tested bacterial strains: 8 strains. | | | |

The results of the determination of the method using Reagent 1 were compared with those of the disk diffusion method (Table 16), and there was 100% agreement for bacteria determined to be susceptible and 80% agreement for bacteria determined to be resistant. These results indicate that the method using Reagent 1 can give a result of drug susceptibility determination that has a high agreement rate with the conventional method in a very short time.

### [Experiment 2] Detection of microorganisms

### 1. Preparation of the reagent of the present invention

The same reagent was prepared as in Reagent 1 used in Experiment 1, except that benzalkonium chloride (bactericide) was used as a cell processing agent at a concentration of 250 mg/L (Reagent 2).

### 2. Microorganisms provided for testing

The microorganisms used were *Escherichia coli, Staphylococcus aureus* and *Candida albicans.*

Isolated microorganisms (*E*. *coli, S*. *aureus,* and *C*. *albicans*) were each smeared on Tryptone-soya agar medium, and cultured at 37 °C for 24 hours. Then, the colonies that developed on the medium were picked up and suspended in sterile physiological saline to be approximately McFarland turbidity 2, which was used as the microbial suspension.

### 3. Determination of the presence/absence of microorganisms using Reagent 2

To 1 mL of the product of the present invention, 100 µL of each microbial suspension or sterile physiological saline with no microorganisms suspended therein was inoculated, incubated at 37 °C for 1 hour, and then the absorbance at 450 nm was measured using a microplate reader, and yellowing was determined when the absorbance was equal to or higher than 0.1 Abs.

### 4. Results

The results are shown in Table 17 (the absorbance of each microbial suspension or the sterile physiological saline when being reacted with the developed product).

**[Table 17]**

| | *E. coli* | *S*. *aureus* | *C. albicans* | Sterile Physiological Saline |
|---|---|---|---|---|
| Absorbance (Abs.) | 0.855 | 0.635 | 0.675 | 0.053 |

All microbial suspensions turned yellow (Table 17), while the sterile physiological saline (negative control) remained colorless (Table 17). These results indicated that the product of the present invention is capable of rapidly determining the presence or absence of a microorganism in a sample.

## Claims

1. A reagent for detecting an intracellular detectable component in a sample, comprising a cell processing agent for transferring the intracellular detectable component to the outside the cell, and a composition for an enzyme cycling reaction for performing the enzyme cycling reaction to the detectable component.

2. The reagent according to Claim 1, wherein the cell is a bacterial or fungal microbial cell.

3. The reagent according to Claim 2, for determining the susceptibility of the microbial cell to the cell processing agent or for determining the presence or absence of the microbial cell in the sample.

4. The reagent according to any one of Claim 3, wherein the cell processing agent is one or more selected from the group consisting of antimicrobial agents, benzalkonium chloride, cetyltrimethylammonium bromide, and cetyltrimethylammonium chloride.

5. The reagent according to Claim 4, wherein the antibacterial agent is one or more selected from the group consisting of cephem antimicrobial agents, penem antimicrobial agents, quinolone antimicrobial agents, penicillin antimicrobial agents, glycopeptide antimicrobial agents, and aminoglycoside antimicrobial agents.

6. The reagent according to Claim 1, wherein the detectable component is one or more selected from the group consisting of NAD+, NADP+, NADH and NADPH.

7. The reagent according to Claim 6, wherein the composition for enzymatic cycling reaction comprises glucose, glucose dehydrogenase, a substrate of diaphorase and diaphorase.

8. The reagent according to Claim 7, wherein the substrate of diaphorase is a tetrazolium salt.

9. The reagent according to Claim 8, wherein the tetrazolium salt is WST-8, 3-(4,5-dimethyl-2-thiazolyl)-2,5- diphenyltetrazolium bromide or Tetranitro Blue Tetrazolium.

10. The reagent according to Claim 1, further comprising a nonionic surfactant.

11. The reagent according to Claim 3, for determining the susceptibility of a microbial cell to the cell processing agent, further comprising cell culture medium ingredients that either do not contain the detectable component or contains it only at a concentration of 5 µg/L or less.

12. The reagent according to Claim 11, wherein the cell culture medium ingredients are peptone and chloride.

13. A method for detecting an intracellular detectable component in a sample, comprising detecting the detectable component that has been transferred to the outside of the cell by a cell processing agent using an enzymatic cycling reaction within the reagent according to any one of Claims 1 to 12.

14. The method according to Claim 13, wherein the cell is a bacterial, fungal or yeast microbial cell, and the method is for determining the susceptibility of the microbial cell to the cell processing agent or for determining the presence or absence of the microbial cell in the sample.

15. A method for detecting an intracellular detectable component in a sample, wherein the method is for determining the susceptibility of microbial cell to a cell processing agent, and wherein the method comprises detecting the detectable component that has been transferred to the outside the cell by the cell processing agent using an enzyme cycling reaction within the reagent according to Claim 11 or 12.
